(19) Europäisches Patentamt
European Patent Office
Office européen des brevets

(11) Veröffentlichungsnummer: **0 112 280**
**B1**

(12) # EUROPÄISCHE PATENTSCHRIFT

(45) Veröffentlichungstag der Patentschrift: **29.08.90**

(21) Anmeldenummer: **83810514.6**

(22) Anmeldetag: **09.11.83**

(51) Int. Cl.[5]: **A 01 N 25/32,** C 07 D 239/34, C 07 D 239/36, C 07 D 239/38, C 07 D 239/42, C 07 D 407/04, C 07 D 409/04

(54) **2-Aryl-4,6-dihalogenpyrimidine als Gegenmittel zum Schützen von Kulturpflanzen vor durch Herbizide verursachten phytotoxischen Schäden.**

(30) Priorität: **15.11.82 CH 6650/82**

(43) Veröffentlichungstag der Anmeldung:
**27.06.84 Patentblatt 84/26**

(45) Bekanntmachung des Hinweises auf die Patenterteilung:
**29.08.90 Patentblatt 90/35**

(84) Benannte Vertragsstaaten:
**BE CH DE FR GB IT LI NL**

(56) Entgegenhaltungen:
**EP-A-0 055 693**
**US-A-3 707 485**

**CHEMICAL ABSTRACTS, Band 94, 1981, Seite 191, Nr. 115867w, Columbus, Ohio, US; YU. V. KARABANOV u.a.: "Stimulating and inhibiting activity of 4,6-dichloro-5-aminopyrimidine derivatives"**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Klybeckstrasse 141**
**CH-4002 Basel (CH)**

(72) Erfinder: **Brunner, Hans-Georg, Dr.**
**Wannenstrasse 14**
**CH-4415 Lausen (CH)**

(56) Entgegenhaltungen:
**CHEMICAL ABSTRACTS, Band 92, 1980, Seiten 573-574, Nr. 94202s, Columbus, Ohio, US; I.V. BOLDYREV u.a.: "4,6-Dichloro-5-isocyanatopyrimidines"**

**Chem. Soc. Rev. 18, 564 (1979)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 112 280 B1

**Beschreibung**

Die vorliegende Erfindung betrifft 2-Aryl-4,6-dihalogenpyrimidine, welche sich als Gegenmittel zum Schützen von Kulturpflanzen vor durch Herbizide verursachten phytotoxischen Schäden eignen. Dabei werden die 2-Aryl-4,6-dihalogenpyrimidine gleichzeitig oder in kurzer Folge mit dem Herbizid den Kulturpflanzungen appliziert. Man kann auch ein Mittel anwenden, welches sowohl das Herbizid wie das 2-Aryl-4,6-dihalogenpyrimidin enthält oder man kann die Samen oder das Saatgut de Kulturpflanze mit dem 2-Aryl-4,6-dihalogenpyrimidin vorbehandeln (beizen) und nachher die gesäge oder aufgelaufene Kultur mit dem Herbizid behandeln. Die Erfindung betrifft auch Mittel welche die 2-Aryl-4,6-dihalogenpyrimidine enthalten sowie ihre Verwendung.

Es ist bekannt, dass Herbizide aus den verschiedensten Stoffklassen, wie Triazine, Harnstoffderivate, Carbamate, Thiolcarbamate, Halogenacetanilide, Halogenphenoxyessigsäure usw. bei der Anwendung in wirksamer Dosis gelegentlich neben den zu schädigen. Ueberdosen werden oft ungewollt und zufälligerweise appliziert, wenn sich Randzonen beim streifenweisen Spritzen überdecken, sei es durch Windeinwirkung oder durch falsches Einschätzen der Breitenwirkung des Spritzgerätes. Es können klimatische Verhältnisse oder eine Bodenbeschaffenheit vorliegen, so dass die für normale Bedingungen empfohlene Herbizidmenge als Ueberdosis wirkt. Die Qualität des Saatgutes kann bei der Herbizidverträglichkeit auch eine Rolle spielen. Um diesem Problem zu begegnen, sind schon verschiedene Stoffe vorgeschlagen worden, welche befähigt sind, die schädigende Wirkung des Herbizids auf die Kulturpflanze spezifisch zu antagonisieren, d.h. die Kulturpflanze zu schützen, ohne dabei die Herbizidwirkung auf die zu bekämpfenden Unkräuter merklich zu beeinflussen. Dabei hat es sich gezeigt, dass die vorgeschlagenen Gegenmittel sowohl bezüglich der Kulturpflanzen als auch bezüglich des Herbizids und gegebenenfalls auch in Abhängigkeit von der Applikationsart oft sehr artspezifisch wirken, d.h. ein bestimmtes Gegenmittel eignet sich oft nur für eine bestimmte Kulturpflanze und einige wenige herbizide Stoffklassen.

Die 2-Aryl-4,6-dihalogenpyrimidine entsprechen der allgemeinen Formel I

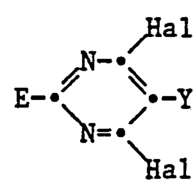

(I)

worin

E eine Gruppe

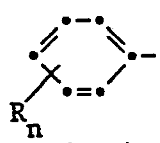 , 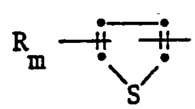 oder 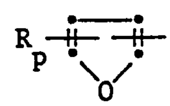 ,

Hal unabhangig voneinander Halogen und

Y ein Gruppe $-NR^1R^2$, $-OR^3$, $-N{=}CR^4R^5$, $-N{=}CH-NR^6R^7$, —SCN oder $-N(R^4)-CN$ bedeuten wobei

R Halogen, Nitro, Cyan, $-XR^8$, $-NR^9R^{10}$, $-CO-R^{11}$, $-COOR^{11}$, $-CO-NR^9R^{10}$, eine unsubstituierte oder durch Halogen, $-XR^8$, substituierte $C_1-C_6$-Alkylgruppe oder eine substituierte oder durch Halogen oder $-XR^8$ substituierte $C_2-C_6$-Alkenyl-, oder $C_2-C_6$-Alkinylgruppe,

n eine Zahl von Null bis drei,

m und p eine Zahl von Null bis zwei,

$R^1$, $R^2$ und $R^3$ unabhängig voneinander Wasserstoff, $-CO-R^4$, $-COOR^4$, $-CON(R^4)_2$, $-CONHR^{12}$, $C_3-C_6$-Alkynyl oder unsubstituiertes oder durch Halogen oder $C_1-C_4$-Alkoxy substituiertes $C_1-C_6$-Alkyl oder $C_3-C_6$-Alkenyl oder

$R^8$ Wasserstoff, $C_1-C_6$-Alkylcarbonyl, oder eine unsubstituierte $C_1-C_6$-Alkyl- gruppe,

wobei auch einer der beiden Reste für $-OR^{11}$, $COOR^{11}$, $-CON(R^{11})$ oder $-CO-N(R^{11})-OR^{11}$ stehen kann,

$R^{11}$ Wasserstoff, eine unsubstituierte oder durch Halogen, substituierte $C_1-C_6$-Alkyl, $C_3-C_6$-Alkenyl oder $C_3-C_6$-Alkinylgruppe,

$R^{12}$ einen Phenylrest

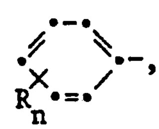 ,

bedeuten.

In den Definition ist unter Alkyl gerakettiges oder verzweigtes Alkyl zu verstehen; z.B.: Methyl, Aethyl, n-Propyl, i-Propyl, die vier isomeren Butyl, n-Amyl, i-Amyl, 2-Amyl, 3-Amyl, n-Hexyl oder i-Hexyl.

Unter Alkoxy ist zu verstehen: Methoxy, Aethoxy, n-Propyloxy, i-Propyloxy und die vier isomeren Butyloxyreste, insbesondere aber Methoxy, Aethoxy oder i-Propoxy.

Beispiele für Alkenylreste sind Vinyl, Allyl, Isoprenyl, 1-Propenyl, 1-Butenyl, 2-Butenyl, 3-Butenyl, 1-Isobutenyl, 2-Isobutenyl, 1-Pentenyl, 2-Pentenyl, 3-Pentenyl und 4-Pentenyl, insbesondere aber Vinyl, Allyl und 4-Pentenyl.

Unter Halogen in den Definitionen sowie in Halogenalkyl, -alkoxy, -alkylsulfinyl, -alkylsulfonyl und -alkylthio sind Fluor, Chlor und Brom, vorzugsweise jedoch Fluor und Chlor zu verstehen.

Alkylinresten in den Definition der obigen Symbole entsprechen in der Regel Propargyl, 2-Butinyl, 3-Butinyl, sowie isomere Pentinyl- oder Hexinylreste, vorzugsweise ist der Alkinylrest jedoch durch Propargyl oder 2- oder 3-Butinyl verkörpert.

Unter Cycloalkyl sind im Rahmen der vorleigenden Anmeldung Cyclopropyl, Cyclobutyl, Cyclopentyl und Cyclohexyl zu verstehen.

Heterocyclen, die von den Resten $R^1$ und $R^2$ bzw. $R^9$ und $R^{10}$, zusammen mit dem Stickstoffatom gebildet werden können sind vorzugsweise Pyrrolidin, Piperidin, Morpholin oder Thiomorpholin.

Unter Halogen als Substituent des Pyrimidinkerns ist Fluor, Chlor oder Brom, insebesondere aber Chlor zu verstehen.

Bevorzugt sind solche Verbindungen, in denen Hal gleichzeitig für zwei g iche Halogensubstituenten steht.

Eine Bevorzugung einiger Wirkstoffe der Formel I liegt darin, dass E für

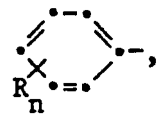

worin R und n die unter Formel I gegebene Bedeutung haben, steht.

Ebenso bevorzugt sind diejenigen Verbindungen der Formel I, in denen E für eine der Gruppen

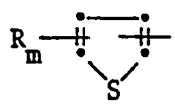 oder 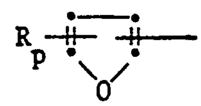

steht, worin R die unter Formel I gegebene Bedeutung hat und m und p Zahlzen von Null bis zwei bedeuten.

Unter den Verbindungen der Formel I, in denen E für die Phenylgruppe steht, sind wiederum die Verbindungen bevorzugt in denen n eine Zahl von Null bis zwei und R Halogen, Nitro, Cyan, $—XR^8$, $NR^9R^{10}$, $—CO—R^{11}$, $—COOR^{11}$, $—CO—NR^9R^{10}$, $C_1—C_4$-Alkyl, $C_2—C_4$-Alkenyl oder $C_2—C_4$-Alkinyl bedeuten, wobei $R^8$, $R^9$, $R^{10}$ und $R^{11}$ die unter Formel I gegebene Bedeutung haben.

Unter den letztgennanten Verbindungen sind diejenigen weiterhin bevorzugt, in denen Hal für Chlor oder Brom steht.

Eine besonders bevorzugte Untergruppe von Verbindungen der Formel I sind die Verbindungen, in denen E für einen unsubstituierten oder einfach durch Methoxy, Fluor, Hydroxyl, Methyl, Aethinyl, Carboxyl, Acetyl, Nitro, Amino, Dimethylamino, Acetamido oder Methylaminocarbonyloxy oder zweifach durch Fluor oder Chlor oder Brom steht und Y für $—NR^1R^2$, $—OR^3$, $—SR^3$, $—N{=}CH—NR^6R^7$ oder $—N{=}CR^4R^5$ steht, wobei $R^1$ bis $R^7$ die unter Formel I gegebene Bedeutung haben.

Insbesondere sind die Verbindungen der Formel I hevorzuheben, in denen E für Phenyl, Hal für Chlor und Y für Amino, Dimethylamino, Diacetamido, Trifluoroacetamido, Hydroxyl, Methoxy, Methylthio, Acetoxy, Acetamido oder Methylaminocarbonyloxy stehen.

Als bevorzugte Einzelverbindungen sind zu nennen:

5-Amino-4,6-dichlor-2-phenylpyrimidin, 4,6-Dichlor-5-methoxy-2-phenylpyrimidin und 4,6-Dichlor-5-hydroxy-5-phenylpyrimidin.

Die 2-Aryl-4,6-dihalogenpyrimidine der Formel I eignen sich hervorragend, Kulturpflanze- wie Hirse, Reis, Mais, Getreidearten (Weizen, Roggen, Gerste, Hafer), Baumwolle Zuckerrüben, Zuckerrohr, Soja etc. vor de phytotoxischen Wirkung von Herbiziden verschiedenster Stoffklassen, wie Triazinen, Phenylharnstoffderivaten, Carbamaten, Thiolcarbamaten, Halogenacetaniliden, Halogenphenoxyesssigsäureestern, substituierte Phenoxyphenoxyessigsäureestern und -propionsäureestern, substituierten Pyridinoxyphenoxy-essigsäureestern und -propionsäureestern, Benzoesäurederivaten usw. zu schützen, sofern diese nicht oder nicht genügend selektiv wirken, also neben den zu bekämpfenden Unkräutern auch die Kulturpflanzen mehr oder weniger schädigen. Die Erfindung betrifft auch Mittel, welche diese 2-Aryl-4,6-dihalogenpyrimidine der Formel I zusammen mit Herbiziden enthalten.

Als Gegenmittel oder Antidote sind schon verschiedene Stoffe vorgeschlagen worden, welche befähigt sind, die schädigende Wirkung eines Herbizides auf die Kulturpflanze spezifisch zu antagonisieren, d.h. die Kulturpflanze zu schützen, ohne dabie die Herbizidwirkung auf die zu bekämpfenden Unkräuter merklich zu beeinflussen. Dabei kann ein solches Gegenmittel, auch Safener genannt, je nach seinen Eigenschaften, zur Vorbehandlung des Saatgutes der Kulturpflanze (Beizung des Samens oder der Stecklinge) oder vor der Saat in die Saatfurchen oder als Tankmischung zusammen mit dem Herbizid vor oder nach dem Auflaufen der Pflanzen verwendet werden.

So beschreibt die GP—PS 1,277,557 die Behandlung von Samen bzw. Sprösslingen von Weizen und Hirse mit gewissen Oxamsäureestern und Amiden vor dem Angriff durch N-Methoxymethyl-2′,6′-diäthyl-

chloracetanilid (Alachlor). In andere Literaturstellen (DE—OS 1,952,910, DE—OS 2,245,471, FR—PS 2,021,611) werden Gegenmittel zur Behandlung von Getreide, Mais- und Reise-Samen zum Schutz gegen den Angriff herbizider Thiolcarbamate vorgeschlagen. In der DE—PS 1,576,676 und der US—PS 3,131,509 werden Hydroxy-amino-acetanilide und Hydantoine für den Schutz von Getreidesamen gegenüber Carbamaten wie IPC, CIPC etc. vorgeschlachen. In der weiteren Entwicklung haben sich alle diese Präparate jedoch als ungenügend erwiesen.

Kürzlich sind in der EP—A—55693 Phenylpyrimidine mit Safener-Wirkung beschriene worden.

Ueberraschenderweise besitzen 2-Aryl-4,6-dihalogenpyrimidine der Formel I die Eigenschaft, Kulturplanzen vor dem Angriff pflanzenaggressiver Agrarchemikalien zu schützen, insbesonderer vor Herbiziden der verschiedensten Stoffklassen wie beispielsweise Chloracetanilide, Chloracetamide, Carbamate und Thiocarbamate, Diphenyläther und Nitrodiphenyläther, Benzoesäurederivate, Triazine und Triazinone, Phenylharnstoffe, Nitroaniline, Oxdiazolone, Pyridyloxyphenoxyderivate, Phosphate und Pyrazole, sofern diese nicht oder ungenügend kulturentolerant sind.

Vorzugweise werden die Kulturpflanzen vor den Herbiziden der Klassen Chloracetanilide, Chloracetamide, Thiocarbamate, Phosphate durch die erfindungsgemässen 2-Aryl-4,6-dihalogenpyrimidine geschützt.

Eine solches Gegenmittel oder Antidote der Formel I kann je nach Anwendungszweck zur Vorbehandlung des Saatgutes der Kulturpflanze (Beizung des Samens oder der Stecklinge) eingesetzt oder vor oder nach der Saat in den Erdboden gegeben weren oder aber für sich allein oder zusammen mit dem Herbizid vor oder nach dem Auflaufen de Pflanzen appliziert werden. Die Behandlung der Pflanze oder des Saatgutes mit den Antidote kann daher grundsätzlich unabhängig vom Zeitpunkt der Applikation der phytotoxischen Chemikalie erfolgen. Sie kann jedoch auch gleichzeitig durchgeführt werden (Tankmischung). Vorauflauf-Behandlung schliesst sowohl die Behandlung der Anbaufläche vor der Aussaat (ppi = "pre plant incorporation") als auch die Behandlung der angesäten, aber noch nicht bewachsenen Anbauflächen ein.

Die Aufwandmengen des Antidotes im Verhältnis zum Herbizid richten sich weitgehend nach der Anwendungsart. Sofern eine Feldbehandlung vorgenommen wird, entweder als Tankmischung oder bei getrennter Applikation von Herbizid und Gegenmittel, verhalten sich die Mengen von Gegenmittel zu Herbizid in der Regel wie 1:100 bis 10:1, bevorzugt wird jedoch der Bereich 1:5 bis 8:1, besonders 1:1.

Bei Samenbeizung und ähnlich gezielten Schutzmassnahmen werden jedoch weit geringere Mengen Gegenmittel im Vergleich mit den z.B. später pro Hektar Anbaufläche verwendeten Herbizidmengen benötigt. Bei der Samenbeizung werden üblicherweise pro kg Samen 0,1—10 g Gegenmittel benötigt, die bevorzugte Menge liegt zwischen 1 und 2 Gramm. Falls das Gegenmittel kurz vor de Aussat durch Samenquellung appliziert werden soll, werden z.B. Gegenmittel-Lösungen, welche den Wirkstoff in einer Konzentration von 1—10 000 ppm enthalten, bevorzugt 100—1000 ppm, verwendet.

In de Regel folgen sich protektive Massnahmen wie Samenbeizung mit einem Gegenmittel der Formel I und mögliche spätere Feldbehandlung mit Agrarchemikalien in zeitlich grösserem Abstand. Vorbehandeltes Saat- und Pflanzengut kann später in Landwirtschaft, Gartenbau und Forstwirtschaft mit unterschiedlichen Chemikalien in Berührung kommen. Die Erfindung bezieht sich daher auch auf kulturpflanzenprotektive Mittel, die als Wirkstoff ein Gegenmittel der Formel I zusammen mit üblichen Trägerstoffen enthalten. Solche Mittel können gegebenfalls zusätzlich mit jenen Agrarchemikalien gemischt sein, vor deren Einfluss die Kulturpflanze geschützt werden soll, z.B. mit einem Herbizid.

Als Kulturpflanzen gelten im Rahmen vorliegender Erfindung alle Pflanzen, die in irgendeiner Form Ertragsstoffe produzieren (Samen, Wurzeln, Stengel, Knollen, Blätter, Blüten, Inhaltstoffe wie Oele, Zucker, Stärke, Eweiss etc.) und zu dienem Zweck angebaut und gehegt werden. Zu diesen Pflanzen gehören beispielsweise sämtliche Getreidearten, Weizen, Roggen, Gerste, Hafer, daneben vor allem Reis, Kulturhirse, Mais, aber auch Baumwolle Zuckerrüben, Zuckerrohr, Soja, Bohnen, Erbsen.

Das Gegenmittel soll überall dort eingesetzt werden, wo ein Kulturpflanze vor der Phytotoxität einer Chemikalie geschützt werden soll.

Als Herbizide, vor deren Wirkung es die Kulturpflanzen zu schützen gilt, seien beispielsweise folgende genannt:

*Chloracetanilide:* 2 - Chlor - 2′,6′ - diäthyl - N - (2″ - propyloxyäthyl) - acetanilid ("Pretilachlor"), 2 - Chlor - 6′ - äthyl - N - (2″ - methoxy - 1″ - methyläthyl) - acet - o - toluidid ("Metolachlor"), 2 - Chlor - 2′,6′ - diäthyl - N - (butoxymethyl) - acetanilid ("Butachlor"), 2 - Chlor - 6′ - äthyl - N - (äthoxymethyl) - acet - o - toluidid ("Acetochlor"), 2 - Chlor - 6′ - äthyl - N - (2″ - propoxy - 1″ - methyläthyl) - acet - o - toluidid, 2 - Chlor - 2′,6′ - dimethyl - N - (2″ - methoxy - 1″ - methyläthyl) - acetanilid, 2 - Chlor - 2′,6′ - dimethyl - N - (2″ - methoxyäthyl) - acetanilid ("Dimethachlor"), 2 - Chlor - 2′,6′ - diäthyl - N - (pyrazol - 1 - ylmethyl) - acetanilid, 2 - Chlor - 6′ - äthyl - N - (pyrazol - 1 - ylmethyl) - acet - o - toluidid, 2 - Chlor - 6′ - äthyl - N - (3,5 - dimethyl - pyrazol - 1 - ylmethyl) - acet - o - toluidid, 2 - Chlor - 6′ - äthyl - N - (2″ - butoxy - 1 - methyläthyl) - acet-o-toluidid ("Metazolachlor"), 2 - Chlor - 6′ - äthyl - N - (2″ - butoxy - 1″ - (methyläthyl) - acet - o - toluidid, 2 - Chlor - 2′ - trimethylsilyl - N - (butoxymethyl) - acetanilid, 2 - Chlor - 2′,6′ - diäthyl - N - (methoxymethyl) - acetanilid ("Alachlor"), 2 - Chlor - 2′,6′ - diäthyl - N - (äthoxycarbonylmethyl) - acetanilid, 2 - Chlor - N - (2″ - n - propoxyäthyl) - 2′,6′ - acetoxylid, 2 - Chlor - 6′ - äthyl - N - (2″ - n - propoxyäthyl) - acet - o - toluidid, 2 - Chlor - N - isopropyl - 3′,3′,5′ - trimethyl - acetanilid, 2 - Chlor -

6' - tert.butyl - N - (n - butoxymethyl) - acet - o - toluidid (Terbluchlor), 2 - Chlor - N - (isobutoxymethyl) - 2',6' - acetoxylid, 2 - Chlor - N - - (sec.butoxymethyl) - 2',6' - acetoxylid.

*Chloracetamide:* N - [ - Isopropyl - 2 - methylpropen - 1 - yl - (1)] - N - (2' - methoxyäthyl) - chloracetamid, 2 - Chlor - N - (n - butoxymethyl) - - 2',6' - dimethyl - 1 - cyclohexenacetamid und N - Isopropyl - 2 - chlor - N - (3,3,5 - trimethyl - 1 - cyclohexen - 1 - yl - chloracetamid.

*Carbamate and Thiocarbamate:* N - (3',4' - Dichlorophenyl) - propionanilid ("Propanil"), S - 4 - Chlorbenzyl - diäthyl - thiocarbamat ("Thiobencarb"), S - Aethyl - N,N - hexamethylen - thiocarbamat ("Molinate"), S - Aethyl - dipropyl - thiocarbamate ("EPTC"), N,N - di - sec.Butyl - S - benzyl - thiocarbamat (Drepamon), S - (2,3 - Dichloroallyl) - di - isopropylthiocarbamat und S(2,3,3 - Trichloroallyl) - di - isopropylthiocarbamat ("Di- und Tri-allate"), 1 - (Propylthiocarbonyl) - decahydro - chinaldin, S - 4 - Benzyldiäthylthiocarbamat sowie entsprechende Sulfinylcarbamate.

*Dimedone:* 2 - [1 - Aethoxyimino) - butyl] - 5 - (äthylthio) - propyl - 3 - hydroxy - 2 - cycylohexen - 1 - on ("Sethoxydin") und das Na-Salz von 2 - [1 - (N - Allyloxamino) - butyliden] - 5,5 - dimethyl - 4 - methoxycarbonyl - cyclohexan - 1,3 - dion ("Alloxidimedon").

*Diphenyläther und Nitrodiphenyläther:* 2,4 - Dichlorphenyl - 4' - nitrophenyläther ("Nitrofen"), 2 - Chlor - 1 - (3' - äthoxy - 4' - nitrophenoxy) - 4 - trifluormethyl - benzol ("Oxyfluorfen"), 2',4' - Dichlorphenyl - 3 - methoxy - äther ("Chlormethoxinyl"), 2 - [4' - (2'',4'' - Dichlorphenoxy) - phenoxy) - propionsäure - methylester, N - (2' - Methoxyäthyl) - 2 - [5' - (2'' - chlor - 4'' - trifluormethylphenoxy) - phenoxy] - propionsäureamid, α - [4 - (2,4 - Dichlorphenoxy) - phenoxy] - propionsäure - methylester (Hoelon), α - [4 - (5' - trifluoromethylpyridyl - 2' - oxy) - phenoxy] - propionsäure - n - butylester (Fluazifop-butyl) und α - [4 - (3',5' - Dichlorpyridyl - 2' - oxy) - phenoxy] - propionsäure - propylester (Chlorazifop-propinyl).

*Benzoesäurederivate:* Methyl - 5 - (2',4' - dichlorphenyl) - 2 - nitrobenzoat ("Bifenox"), 5 - (2' - Chlor - 4' - trifluormethylphenoxy) - 2 - nitrobenzoesäure ("Acifluoren") 2,6 - Dichlorbenzonitril ("Dichlobenil").

*Triazine und Triazinone:* 2,4 - Bis(isopropylamino) - 6 - methylthio - 1,3,5 - triazin ("Prometryn"), 2,4 - Bis(äthylamino) - 6 - methylthio - 1,3,5 - triazin ("Simetryn"), 2 - (1',2' - Dimethylpropylamino) - 4 - äthylamino - 6 - methylthio - 1,3,5 - triazin ("Dimethametryn"), 4 - Amino - 6 - tert.butyl - 4,5 - dihydro - 3 - methylthio - 1,2,4 - triazin - 5 - on ("Metribuzin").

*Phenylharnstoffe:* N - (3' - Isopropylphenyl) - N',N' - dimethyl - harnstoff ("Isoproturon"), N - (3',4' - Dimethylbenzyl) - N' - 4 - tolyl - harnstoff ("Dimuron"), N - (3' - Chlor - 4 - isopropylphenyl) - N',N' - (3 - methyl - pentamethylen - 1,5 - yl) - harnstoff.

*Nitroaniline:* 2,6 - Dinitro - N,N - dipropyl - 4 - trifluormethylanilin ("Trifluralin"), N - (1' - Aethylpropyl) - 2,6 - dinitro - 3,4 - xyliden ("Pendimethalin").

*Oxadiazolone:* 5 - tert. - Butyl - 3 - (2',4' - dichloro - 5' - isopropoxyphenyl) - 1,3,4 - oxadiazol - 2 - on ("Oxidazon").

*Pyridyloxyphenoxyderivate:* 2 - [4' - (3'',5'' - Dichlorpyridyl - 2'' - oxy) - phenoxy] - propionsäure - (2 - propinyl)ester.

*Phosphate:* S - 2 - Methylpiperidino - carbonylmethyl - O,O - dipropyl - phosphorodithioat ("Piperophos").

*Pyrazole:* 1,3 - Dimethyl - 4 - (2',4' - dichlorobenzoyl) - 5 - (4' - tolylsulfonyloxy) - pyrazol.

*Diverse* 2 - Aethoxy - 2,3 - dihydro - 3,3 - dimethylbenzofuran - 5 - yl - methan sulfonat ("Ethofumesat"), Benzthiazol - (2) - yl - oxy - essigsäure - N - methylanilid.

Das 2-Aryl-4,6-dihalogenpyrimidin der Formel I oder das Mittel, welches dieses Gegenmittel enthält kann wahlweise vor oder nach der Applikation des Herbizides oder auch gleichzeitig mit diesem angewendet werden. Besonders rationell hat sich das Behandeln der Samen durch eine das Gegenmittel enthaltende Lösung (Samenbeizung) erwiesen. Dabei kann das Lösungsmittel verdunstet werden und der Samen trocken, mit einem Gegenmittel-Belag behafter zur Anwendung kommen oder man kann den Samen in einer wässerigen, das Gegenmittel enthaltend Lösung vorquellen und in diesem Zustand aussäen wie es bespielsweise bei Reise üblich ist.

Die 2-Aryl-4,6-dihalogenpyrimidine der Formel I werden in sich bekannter Weise erhalten, indem man eine Arylamidin der Formel II

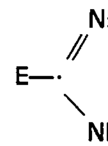

(II),

worin E unter Formel I gegebene Bedeutung hat, in Gegenwart einer Base mit einem Malonsäurederivat der Formel III

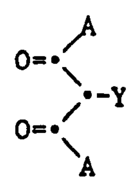

(III),

worin Y die unter Formel I gegebene Bedeutung hat und A für Amino oder $C_1$—$C_6$-Alkoxy steht, umsetzt und das erhaltene, 4,6-Dihydroxypyrimidin der Formel IV

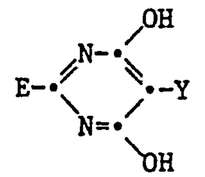

(IV),

worin E und Y die unter Formel I gegebene Bedeutung haben, mit einem Halogenierungsmittel in die Verbindung der Formel I Überführt.

Analoge Verfahren sind aus der europläischen Patentpublikation 55693 bekannt.

Als geeignete Basen seien genannt: Hydroxide wie NaOH, KOH; Oxide wie CaO, MgO; Carbonate wie $Na_2CO_3$, $K_2CO_3$, $CaCO_3$, $MgCO_3$; Hydrogencarbonate wie $NaHCO_3$, $KHCO_3$, $Ca(HCO_3)_2$; Alkoholate wie $NaOCH_3$, $NaOC_2H_5$, $NaOC_4H_9$-t, $NaOC_3H_7$-i, $KOCH_3$, $KOC_2H_5$, $KOC_4H_9$-t, $KOC_3H_7$-i. Beispiele für Halogenierungsmittel sind $SOCl_2$, $SOBr_2$, $PCl_3$, $PBr_3$, $PCl_5$, $PBr_5$, $POCl_3$, $POBr_3$. Das erfindungsgemässe Verfahren kann in beiden Stufen bei Temperaturen zwischen 0° und 150°C, vorzugsweise zwischen 20° und 120°C durchgeführt werden. Besonders vorteilhaft ist für die Durchführung der Reaktion ein Erhitzen des Gemisches bis zum Rückluss. Die erste Reaktionsstufe wird mit Vorteil in einem interten Lösungsmittel ausgeführt, die zweite dagegen meist ohne Lösungsmittel, sofern das Chlorierungsmittel flüssig ist und im Ueberschuss eingesetzt werden kann. Geeignete Lösungsmittel für die erste Stufe sind Alkohole wie Methanol, Aethanol, Propanol, Isopropanol; Kohlenwasserstoffe wie Benzol, Toluol, Xylol, Aether wie Diäthyläther, Aethylenglykoldimethyläther, Diäthylenglykoldimethyläther, Dioxan, Tetrahydrofuran; Ketone wie Aceton, Aethylmethylketon, Cyclohexanon, oder Dimethylsulfoxid, insbesondere jedoch Alkohole wie Methanol und Aethanol. Geeignete Lösungsmittel fuř die zweite Stufe sind Kohlenwasserstoffe wie Benzol, Toluol, Xylol, Cyclohexan sowie die verschiedenen Benzinfraktionen oder die vorgenannten Aether.

Einzelne Untergruppen der Verbindungen der Formel I sind nach an sich bekannten Verfahren aus einzelnen Schlüsselverbindungen herstellbar. So kann mit die Verbindungen der Formel I, in denen Y für die Gruppen $—NR^1R^2$, $—N=CR^4R^5$, $—N=CH—NR^6R^7$ oder $—N(R^4)—CN$ steht, wenn man die zunächst nach den Schemata 1 und 2 hergestellte Aminoverbindung nach allgemein bekannten Methoden in die übrigen Derivate überführt.

Schema 1:

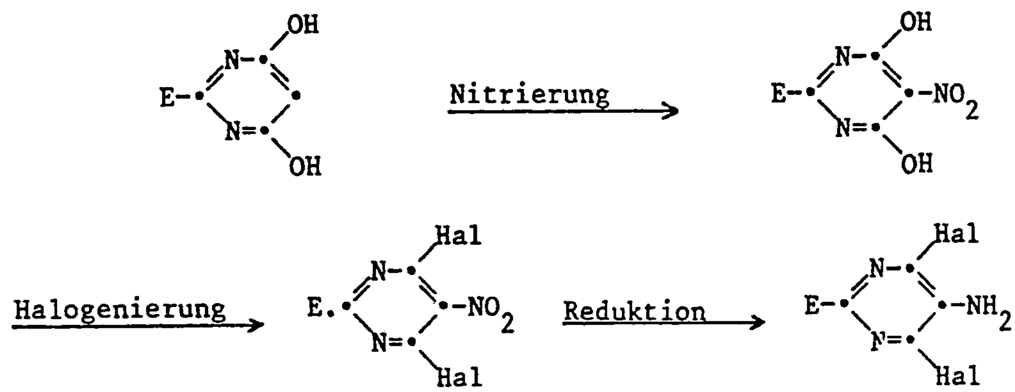


Schema 2:

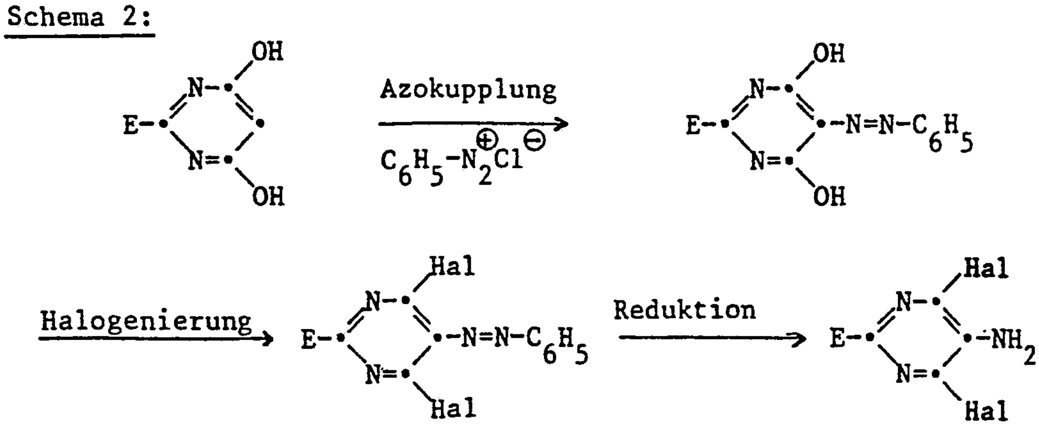

Die Verbindung der Formel I, in denen Y für $—OR^3$,$—SR^3$, $—SOR^4$, $SO_2R^4$ oder —SCN steht, kann man durch Ueberführung von entsprechenden Hydroxyl- und Mercaptoverbindungen nach allgemein bekannten Methoden in die genannten Derivate erhalten. Die 5-Hydroxyl- und 3-Mercaptopyrimidine der Formel I sind ihrerseits durch Aetherspaltung aus den entsprechenden 5-Alkoxy- und 5-Alkylthioverbindungen herstellbar.

Die 2-Aryl-4,6-dihalogenpyrimidine der Formel I können für sich allein oder zusammen mit den zu antagonisierenden Herbiziden verwendet werden.

Dabei werden Verbindungen der Formel I in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünntens Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gebenenfalls einen festen oder flüssigen Zusatzstoff enthaltenden Mittel, Zubereintungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoff wie Cyclohexan oder Paraffine, Alkohole und Glykole sowie deren Aether und Ester, wie Aethanol, Aethylenglykol, Aethylenglykolmonomethyl- oder äthyläther, Ketone wie Cyclohexanon, stark polare Lösungsmittel wie N-Methyl-2-pyrrolidon, Dimethylsulfoxid oder Dimethylformamid, sowie gegebenenfals epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägersmaterialien z.B. Calcit oder Sand in Frage. Darüberhinaus kann eine Vielzahl von vorgranulierten Materialien anorganischer oder organischer Nature wie insbesondere Dolomit oder zerkleinerte Pflanzenrückstände verwendet werden.

Als oberflächenaktive Verbindungen kommen je nach der Art dues zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C^1_0$—$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methy-taurinsalze zu erwähnen.

Häufiger erden jedoch soz. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vir und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8—22 C-Atomen. Alkylarylsulfonate sind z.B. die Na-, Ca- oder Triäthanolaminsalze der Dodecylbenzosulfonsäure, der Dibutylnaphthalinsäure, oder eines Naphthalinsulfonsäure-Formaldehydkondensationsproduktes.

Ferner kommen nach entsprechende Phosphate, wie. z.B Salze des Phosphorsäureesters eines p-Nonylphenol-(4-14)-Aethylenoxid-Adduktes oder Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole,

Ricinussölpolyglykoläther, Polypropylen-Polyäthylenoxyaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammomiumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)äthylammoniumbromid.

Die in Der Formulierungstechnik gebräuchlichen Tenside sind u.a. in folgenden Publikation beschrieben:

"McCutcheon's Detergents and Emulsifiers Annual" MC Publishing Corp., Ridgewood, New Jersey, 1979, M. and J. Ash, "Encyclopedia of Surfactants" Vol I—III, Chemical Publishing Co., Inc. New York 1980—1981. H. Stache, "Tenside Taschenbuch" 2. Auflage C. Hauser Verlage, München und Wien, 1981.

Die pestiziden Zubereitungen enthalten in der Regel 0,1 bis 99%, insbesondere 0,1 bis 95%, Wirkstoff der Formel I, 1 bis 99% eines festen oder flüssigen Zusatzstoffes und 0 bis 25%, insbesondere 0,1 bis 25%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel, sowie Dünger oder andere Wirkstoffe zur Erzielung spezieller Effekte enthalten.

In den nachfolgenden Beispielen sind die Temperaturen in Celsiusgraden angegeben, Prozente und Angaben von "Teilen" beziehen sich auf das Gewicht.

## Herstellungsbeispiele:

### Beispiel 1
5-Amino-4,6-dichloro-2-phenylpyrimidin

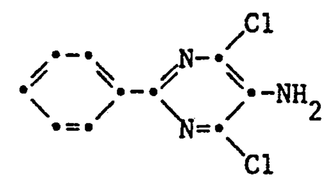

a) 500 ml konzentrierter Salpetersäure werden bei 0°C innerhalb von 12 Minuten mit 147 g 4,6-Dihydroxy-2-phenylpyridin versetzt. Die Reaktionslösung wird für weitere 10 Minuten bei 0°C gerührt und anschliessend in 2 Liter Eiswasser aufgenommen. Das ausgefallene Produkt wird abgetrennt, mit Wasser gewaschen und getrocknet. Man erhält so 158,5 g 4,6-Dihydroxy-5-nitro-2-phenylpyrimidin, Smp. 328—333°C.

b) Eine Mischung von 46,6 g 4,6-Dihydroxy-5-nitro-2-phenylpyrimidin, 48 ml, N,N-Dimethylanilin, 42 ml Phosphoroxychlorid und 270 ml Toluol wird für 40 Minuten zum Rückfluss erhitzt. Danach wird das überschüssige Lösungsmittel abgedampft, der Rückstand in Methylenchlorid aufgenommen und zweimal mit Eiswasser gewaschen. Nach Trocknen und Einengen der organischen Phase erhält man 41,0 g 4,6-Dichlor-5-nitro-2-phenylpyrimidin.

c) 27,0 g 4,6-Dichlor-5-nitro-2-phenylpyrimidin werden in Aethylacetat bei Raumtemperatur durch Behandlung mit Raney-Nickel hydriert. Nach dem Abtrennen des Katalysators wird die Reaktionslösung eingedampft. Durch Kristallisation des Rückstands aus Petroläther erhält man 17,4 g 5-Amino-4,6-dichlor-2-phenylpyrimidin, Smp. 143—144°C (Verbindung Nr. 1).

### Beispiel 2
4,6-Dichlor-5-dimethylamino-2-phenylpyrimidin

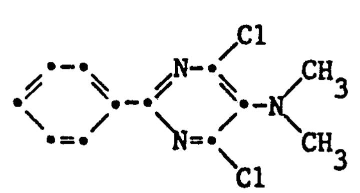

a) Zu einer Lösung von 30,5 g 2-Dimethylaminomalonsäurediäthylester und 20,0 g Benzamidin in 80 ml Methanol lässt man 72,0 ml einer 30%igen Natriummethylatlösung zutropfen und kocht das Gemisch für 6 Stunden am Rückfluss. Nach dem Abdampfen des Lösungsmittels wird der Rückstand in 700 ml Wasser aufgenommen und mit Toluol gewaschen. Die wässrige Phase wird mit Eisessig auf einen pH Wert von 5 angesäuert, das ausgefallene Produkt abgetrennt, gewaschen und getrocknet. Man erhält so 24,3 g 5-Dimethylamino-4,6-dihydroxy-2-phenylpyrimidin, Smp. >295°C.

b) 6,9 g 5-Dimethylamino-4,6-dihydroxy-2-phenylpyrimidin werden in 25 ml Phosphoroxychlorid für 2,5 Stunden am Rückfluss gekocht. Nach dem Abdestillieren des überschüssigen Phosphoroxychlorids wird der Rückstand in einem Aethylacetat/Eiswasser-Gemisch aufgenommen und mit Natriumhydrogencarbonat bis auf einen pH-Wert von 6—7 neutralisiert. Die organische Phase wird mit Wasser gewaschen, getrocknet und eingedampft. Man erhält so 4,2 g 4,6-Dichlor-5-dimethylamino-2-phenylpyrimidin, Smp. 49—50°C (Verbindung Nr. 3).

Beispiel 3
4,6-Dichlor-5-methoxy-2-phenylpyrimidin

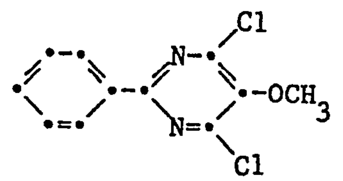

a) Eine Lösung von 20,5 ml Benzonitril in 50 ml Methanol wird bei 0—15°C mit gasförmigem Chlorwasserstoff gesättigt und bei 20—25°C für 18 Stunden gerührt. Anschliessend wird der überschüssige Chlorwasserstoff mit Stickstoffgas vertrieben und der Lösung tropfenweise 50 ml 10 N methanolischer Ammoniaklösung zugesetzt. Nachdem die Mischung für 1 Stunde zum Rückfluss erhitzt worden ist, werden 26,4 g 2-Methoxymalonsäurediamid und 108 ml 30%iger Natriummethylatlösung zugesetzt und das Gemisch wird nochmals für 6 Stunden am Rückfluss gekocht. Die Mischung wird eingeengt, der Rückstand in 250 ml Wasser gelöst und mit Methylenchlorid gewaschen. Nach dem Ansäuren mit Essigsäure wird das ausgefallene Produkt abgetrennt, gewaschen und getrocknet. Man erhält 23,2 g 4,6-Dihydroxy-5-methoxy-2-phenylpyrimidin, Smp. >265°C (Zers.).

b) Eine Gemisch von 10,9 g 4,6-Dihydroxy-5-methoxy-2-phenylpyrimidin, 12,6 ml N,N-Dimethylanilin, 10 ml Phosphoroxychlorid und 50 ml Toluol wird für 2 Stunden zum Rückfluss erhitzt. Nach dem Einengen des Gemisches wird der Rückstand in Aethylacetat aufgenommen, zweimal mit 1N Salzsäure und zweimal mit Wasser gewaschen, getrocknet und eingedampft. Man erhält 10,2 g 4,6-Dichlor-5-methoxy-2-phenylpyrimidin, Smp. 78—80·C (Verbindung Nr. 31).

Beispiel 4
4,6-Dichlor-5-hydroxy-2-phenylpyrimidin

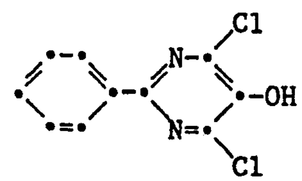

In einem Bombenrohr werden 15,3 g 4,6-Dichlor-5-methoxy-2-phenylpyrimidin und 30 ml 25%iger Bortrichloridlösung in Methylenchlorid für 15 Stunden auf 40°C erwärmt. Anschliessend wird die Reaktionsmischung tropfenweise einem Gemisch von Methanol und Methylenchlorid (1:1) zugesetzt und anschliessend mit Eiswasser verdünnt. Die organische Phase wird abgetrennt, die wässrige mit Methylenchlorid extrahiert. Die vereinigten organischen Phasen werden eingedampft und an Kieselgel chromatographiert. Men erhält so 8,7 g 4,6-Dichlor-5-hydroxy-2-phenylpyrimidin, Smp. 138—140°C (Verbindung Nr. 34).

Beispiel 5
5-Acetoxy-4,5-dichlor-2-phenylpyrimidin

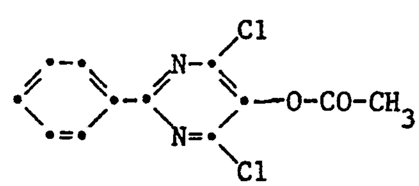

Eine Lösung von 3,1 g 4,6-Dichlor-5-hydroxy-2-phenylpyrimidin, 2,8 ml Trimethylamin, 1,6 ml Essigsäureanhydrid und 0,1 g 4-Dimethylaminopyrimidin in 50 ml Toluol wird für 4 Stunden bei 20—25°C gerührt. Anschliessend wird die Reaktionslösung mit Aethylacetat verdünnt, mit Wasser gewaschen und getrocknet. Beim Einengen kristallisieren 4,3 g 5-Acetoxy-4,6-dichloro-2-phenylpyrimidin aus, Smp. 145—147°C (Verbindung Nr. 36).

In analoger Weise erhält man die in der anschliessenden Tabelle aufgelisteten Endprodukte.

Tabelle 1:

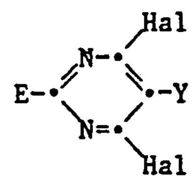

| Nr. | E | Y | Hal | Phys. Daten |
|---|---|---|---|---|
| 1 | $C_6H_5$ | $NH_2$ | Cl, Cl | Smp. 143-144°C |
| 2 | $C_6H_5$ | $-NH-CH_3$ | Cl, Cl | Smp. 71-72°C |
| 3 | $C_6H_5$ | $-N(CH_3)_2$ | Cl, Cl | Smp. 49-50°C |
| 4 | $C_6H_5$ | $-NH-CH_2-CH=CH_2$ | Cl, Cl | |
| 5 | $C_6H_5$ | N-Pyrrolidinyl | Cl, Cl | Smp. 68-73°C |
| 6 | $C_6H_5$ | $-NH-(CH_2)_2-OCH_3$ | Cl, Cl | |
| 7 | $C_6H_5$ | $-NH-(CH_2)_2-Cl$ | Cl, Cl | |
| 8 | $C_6H_5$ | $-NH-CH_2-C\equiv CH$ | Cl, Cl | |
| 9 | $C_6H_5$ | -NH-CN | Cl, Cl | |
| 10 | $C_6H_5$ | $-NH-CO-CH_3$ | Cl, Cl | Smp. 216-218°C |
| 11 | $C_6H_5$ | $-N(COCH_3)_2$ | Cl, Cl | Smp. 104-106°C |
| 12 | $C_6H_5$ | $-NH-CO-CH_2Cl$ | Cl, Cl | |
| 13 | $C_6H_5$ | $-NH-CO-CF_3$ | Cl, Cl | Smp. 174-177°C |
| 14 | $C_6H_5$ | $-NH-CO-CH=CH_2$ | Cl, Cl | |
| 15 | $C_6H_5$ | $-NH-CO-NH-CH_3$ | Cl, Cl | |
| 16 | $C_6H_5$ | $-NH-CO-NH-C_4H_9-n$ | Cl, Cl | |
| 17 | $C_6H_5$ | $-NH-CO-N(CH_3)_2$ | Cl, Cl | |
| 18 | $C_6H_5$ | $-NH-CO-N(CH_3)OCH_3$ | Cl, Cl | |
| 19 | $C_6H_5$ | $-N(CH_3)-CO-N(CH_3)OCH_3$ | Cl, Cl | |
| 20 | $C_6H_5$ | $-NH-CO-OCH_3$ | Cl, Cl | |
| 21 | $C_6H_5$ | $-NH-CO-O-CH=CH_2$ | Cl, Cl | |
| 22 | $C_6H_5$ | $-N(CH_3)-CO-OCH_3$ | Cl, Cl | |
| 23 | $C_6H_5$ | $-NH-SO_2-CH_3$ | Cl, Cl | |
| 24 | $C_6H_5$ | $-NH-SO_2-CF_3$ | Cl, Cl | |
| 25 | $C_6H_5$ | $-N(CH_3)-CO-CF_3$ | Cl, Cl | Smp. 135-137°C |

Tabelle 1 (Fortsetzung)

| Nr. | E | Y | Hal | Phys. Daten |
|---|---|---|---|---|
| 26 | $C_6H_5$ | $-N=CH-N(CH_3)_2$ | Cl, Cl | |
| 27 | $C_6H_5$ | $-N=CH-CH_3$ | Cl, Cl | |
| 28 | $C_6H_5$ | $-N=CH-C_3H_7$-i | Cl, Cl | |
| 29 | $C_6H_5$ | -NH-CHO | Cl, Cl | |
| 30 | $C_6H_5$ | $-NH-C_3H_7$-i | Cl, Cl | |
| 31 | $C_6H_5$ | $-OCH_3$ | Cl, Cl | Smp. 78-80°C |
| 32 | $C_6H_5$ | $-O-CH_2-CH=CH_2$ | Cl, Cl | |
| 33 | $C_6H_5$ | $-O-CH_2-C\equiv CH$ | Cl, Cl | |
| 34 | $C_6H_5$ | OH | Cl, Cl | Smp. 138-140°C |
| 35 | $C_6H_5$ | $-O-CH_2-CH_2Cl$ | Cl, Cl | |
| 36 | $C_6H_5$ | $-O-CO-CH_3$ | Cl, Cl | Smp. 145-147°C |
| 37 | $C_6H_5$ | $-O-CO-CH_2Cl$ | Cl, Cl | |
| 38 | $C_6H_5$ | $-O-CO-C_2H_5$ | Cl, Cl | |
| 39 | $C_6H_5$ | $-O-CO-C_5H_{11}$-n | Cl, Cl | |
| 40 | $C_6H_5$ | $-O-CO-CH=CH-CH_3$ | Cl, Cl | |
| 41 | $C_6H_5$ | $-O-CO-C_4H_9$-i | Cl, Cl | |
| 42 | $C_6H_5$ | $-O-CO-NH-CH_3$ | Cl, Cl | Smp. 240°C (Zers.) |
| 43 | $C_6H_5$ | $-O-CO-NH-C_4H_9$-n | Cl, Cl | |
| 44 | $C_6H_5$ | $-O-CO-N(CH_3)_2$ | Cl, Cl | |
| 45 | $C_6H_5$ | $-O-CO-N(CH_3)OCH_3$ | Cl, Cl | |
| 46 | $C_6H_5$ | $-O-SO_2-CH_3$ | Cl, Cl | |
| 47 | $C_6H_5$ | $-O-SO_2-CF_3$ | Cl, Cl | |
| 48 | $C_6H_5$ | $-O-CO-OCH_3$ | Cl, Cl | |
| 49 | $C_6H_5$ | $-SCH_3$ | Cl, Cl | Smp. 96-98°C |
| 50 | $C_6H_5$ | $-S-CO-CH_3$ | Cl, Cl | |
| 51 | $C_6H_5$ | -S-CN | Cl, Cl | |
| 52 | $C_6H_5$ | SH | Cl, Cl | |
| 53 | $C_6H_5$ | $-S-CO-N(CH_3)-OCH_3$ | Cl, Cl | |
| 54 | $C_6H_5$ | $-S-C_3H_7$-n | Cl, Cl | |

Tabelle 1 (Fortsetzung)

| Nr. | E | Y | Hal | Phys. Daten |
|---|---|---|---|---|
| 55 | $4-OCH_3-C_6H_4-$ | $NH_2$ | Cl, Cl | |
| 56 | $3-OCH_3-C_6H_4-$ | $N(CH_3)_2$ | Cl, Cl | |
| 57 | $4-F-C_6H_4-$ | $OCH_3$ | Cl, Cl | |
| 58 | $4-F-C_6H_4-$ | OH | Cl, Cl | |
| 59 | $4-F-C_6H_4-$ | $O-CO-CH_3$ | Cl, Cl | |
| 60 | $3-OCH_3-C_6H_4-$ | $OCH_3$ | Cl, Cl | Smp. 113-115°C |
| 61 | $3-OH-C_6H_4-$ | OH | Cl, Cl | |
| 62 | $3-CH_3-C_6H_4-$ | $NH_2$ | Cl, Cl | |
| 63 | $3-CH_3-C_6H_4-$ | $NH-CO-CH_3$ | Cl, Cl | |
| 64 | $3-CH_3-5-CH_3-C_6H_3-$ | $NH-CH_3$ | Cl, Cl | |
| 65 | $3-CH_3-5-CH_3-C_6H_3-$ | $NH-CO-OCH_3$ | Cl, Cl | |
| 66 | $4-HC{\equiv}C-C_6H_4-$ | $OCH_3$ | Cl, Cl | |
| 67 | $3-Cl-C_6H_4-$ | $-NH-CH_2-C{\equiv}CH$ | Cl, Cl | |
| 68 | $3-Cl-4-F-C_6H_3-$ | $SCH_3$ | Cl, Cl | |
| 69 | $4-HOOC-C_6H_4$ | $NH_2$ | Cl, Cl | |
| 70 | $4-CH_3-CO-C_6H_4-$ | $-N(CH_3)_2$ | Cl, Cl | |
| 71 | $3-NO_2-C_6H_4-$ | $OCH_3$ | Cl, Cl | |
| 72 | $3-NO_2-C_6H_4$ | OH | Cl, Cl | |
| 73 | $3-NH_2-C_6H_4-$ | OH | Cl, Cl | |
| 74 | $3-CH_3-CO-NH-C_6H_4-$ | $-O-CO-CH_3$ | Cl, Cl | |
| 75 | $4-OH-C_6H_4$ | OH | Cl, Cl | |
| 76 | $4-OCH_3-C_6H_4-$ | $OCH_3$ | Cl, Cl | |
| 77 | $4-CH_3-NH-CO-O-C_6H_4-$ | $-O-CO-NH-CH_3$ | Cl, Cl | |
| 78 | $3-OCH_3-4-OCH_3-5-OCH_3-C_6H_2-$ | $NH_2$ | Cl, Cl | |
| 79 | $3-OCH_3-4-OCH_3-5-OCH_3-C_6H_2-$ | $-NH-SO_2-CF_3$ | Cl, Cl | |
| 80 | $3-Cl-4-Cl-C_6H_3-$ | $-O-C_4H_9-n$ | Cl, Cl | |
| 81 | $4-N(CH_3)_2-C_6H_4-$ | $-O-CO-CH_2Cl$ | Cl, Cl | |
| 82 | $2-OCH_3-C_6H_4-$ | N-Pyrrolidinyl | Cl, Cl | |
| 83 | $C_6H_5$ | OH | Br, Br | |

Tabelle 1 (Fortsetzung)

| Nr. | E | Y | Hal | Phys. Daten |
|---|---|---|---|---|
| 84 | $C_6H_5$ | $NH_2$ | Br, Br | Smp. 126-129°C |
| 85 | 4-Tolyl | OH | Br, Br | |
| 86 | $C_6H_5$ | OH | Br, Cl | |
| 87 | $C_6H_5$ | $OCH_3$ | Br, Br | Smp. 88- 89°C |
| 88 | $4-F-C_6H_4-$ | $NH_2$ | Br, Br | |
| 89 | $C_6H_5$ | $NH_2$ | F, Cl | |
| 90 | $4-CH_3-C_6H_4-$ | $-O-CO-CH_3$ | Br, Br | |
| 91 | 2-Thienyl | $NH_2$ | Cl, Cl | |
| 92 | 3-Thienyl | OH | Cl, Cl | |
| 93 | 2-Furyl | $NH_2$ | Cl, Cl | Smp. 139-140°C |
| 94 | 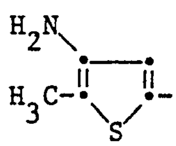 | $NH_2$ | Cl, Cl | Smp.160° (Z) |
| 95 | 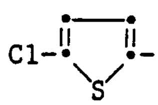 | $-O-CO-NH-CH_3$ | Cl, Cl | |
| 96 | 3-Furyl | $-N(CH_3)_2$ | Cl, Cl | |
| 97 | 3-Thienyl | $OCH_3$ | Br, Br | |
| 98 | 2-Furyl | $OCH_3$ | Cl, Cl | |
| 99 | 2-Furyl | OH | Cl, Cl | |
| 100 | 2-Furyl | $OCH_3$ | Cl, Cl | Smp. 109-111°C |
| 101 | $C_6H_5$ | $-N(CONHC_6H_5)_2$ | Cl, Cl | amorph<br>Smp. > 230°C |
| 102 | 4-Tolyl | $OCH_3$ | Cl, Cl | Smp. 109-111°C |
| 103 | 4-Tolyl | $-NH_2$ | Cl, Cl | Smp. 150-152°C |
| 104 | $4,6-(Cl)_2C_6H_3-$ | $NH_2$ | Cl, Cl | Smp. 199°C |
| 105 | $3-OCH_3C_6H_4-$ | $NH_2$ | Cl, Cl | fest |
| 106 | 4-Tolyl | OH | Cl, Cl | Smp. 133-135°C |
| 107 | $3-ClC_6H_4-$ | $NH_2$ | Cl, Cl | Smp. 168-170°C |
| 108 | $4-ClC_6H_4-$ | $OCH_3$ | Cl, Cl | Smp. 136° |

Tabelle 1 (Fortsetzung)

| Nr. | E | Y | Hal | Phys. Daten |
|---|---|---|---|---|
| 109 | $C_6H_5$ | $N(C_2H_5)_2$ | Cl, Cl | $n_D^{27}$ 1.6012 |
| 110 | $4\text{-}CH_3\text{-}3NO_2C_6H_3\text{-}$ | $NH_2$ | Cl, Cl | Smp. 212-214°C |
| 111 | $4\text{-}CH_3\text{-}3NH_2\text{-}C_6H_5$ | $NH_2$ | Cl, Cl | Smp. 167-168°C |

Formulierungsbeispiele

Die Verbindungen der Formel I werden im allgemeinen nicht als solche in der Landwirtschaft eingesetzt. Man verwendet gebrauchsfertige formulierte Mittel, welche entweder direkt oder mit Wasser verdünnt eingesetzt werden können.

Beispiel 6

Stäubemittel

Zur Herstellung eines a) 5%igen und b) 2%igens Stäubemittels werden die folgenden Stoffe verwendet:

a) 5 Teile 5-Amino-4,6-dichlor-2-phenylpyrimidin oder einer Mischung davon mit 2-Chlor-2',6'-diäthyl-N-(butoxymethyl)-acetanilid,

95 Teile Talkum.

b) 2 Teile des obigen Wirkstoffes oder einer Mischung,

1 Teil hochdisperse Kieselsäure,

97 Teile Talkum.

Die Wirkstoffe werdeb mit den Trägerstoffen vermischt und vermahlen und können in dieser Form zur Anwendung verstäubt werden.

Beispiel 7

Granulat

Zur Herstellung eines 5%igen Granulates werden die folgenden Stoffe verwendet:

5 Teile 4,6-Dichlor-5-hydroxy-2-phenylpyrimidin oder einer Mischung davon mit 2-Chlor-2',6'-diäthyl-N-(methoxymethyl)-acetanilid,

0,25 Teile epoxidiertes Pflanzenöl,

0,25 Teile Cetylpolyglykoläther,

3,50 Teile Polyäthylenglykol,

91 Teile Kaolin (Korngrösse 0,3—0,8 mm).

Die Aktivsubstanz oder die Mischung wird mit dem Pflanzenöl vermischt und mit 6 Teilen Aceton gelöst, hierauf wird Polyäthylenglykol und Cetylpolyglykoläther zugesetzt. Die so erhaltene Lösung wird auf Kaolin aufgesprüht, und anschliessend wird das Aceton im Vakuum verdampft. Ein derartiges Mikrogranulat lässt sich vorteilhaft in Saatfurchen einarbeiten.

Beispiel 8

Spritzpulver

Zur Herstellung eines a) 70%igen, b) 40%igen, c) und d) 25%igen, e) 10%igen Spritzpulvers werden folgende Bestandteile verwendet:

a) 70 Teile 4,6-Dichlor-5-methoxy-2-phenylpyrimidin oder einer Mischung davon mit 2-Chloro-2',6'-diäthyl-N-(2''-propoxyäthyl)-acetanilid,

5 Teile Natriumdibutylnaphthylsulfonat,

3 Teile Naphthalinsulfonsäuren-Phenolsulfonsäuren-Formaldehyd-Kondensat 3:2:1,

10 Teile Kaolin,

12 Teile Champagne-Kreide;

b) 40 Teile Wirkstoff oder Mischung wie oben,

5 Teile Ligninsulfonsäure-Natriumsalz,

1 Teil Dibutylnaphthalinsulfonsäure-Natriumsalz,

54 Teile Kieselsäure,

c) 25 Teile Wirkstoff oder Mischung wie oben,

4,5 Teile Calcium-Ligninsulfonat,

1,9 Teile Champagne-Kreide/Hydroxyäthylcellulose-Gemisch (1:1),

1,5 Teile Natrium-dibutyl-naphthalinsulfonat,

19,5 Teile Champagne-Kreide,

28,1 Teile Kaolin;

d) 25 Teile Wirkstoff oder Mischung wie oben,

2,5 Teil Isooctylphenoxy-polyoxyäthylenäthanol,

1,7 Teile Champagne-Kreide/Hydroxyäthylcellulose-Gemisch (1:1),

8,3 Teile Natriumaluminiumsilkat,

16,5 Teile Kieselgur,

46 Teile Kaolin;

e) 10 Teile Wirkstoff oder Mischung wie oben,

3 Teile Gemisch der Natriumsalze von gesättigten Fettalkoholsulfaten,

5 Teile Naphthalinsulfonsäure/Formaldehyd-Kondensat,

82 Teile Kaolin.

Die Wirkstoffe werden in geeigneten Mischern mit den Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnen und insbesondere zur Blattapplikation (zwecks Wuchsverzögerung oder für Fungizideinsatz) verwenden lassen.

Beispiel 9

Emulgierbare Konzentrate

Zur Herstellung eines 25%igen emulgierbaren Konzentrates werden folgende Stoffe verwendet:

25 Teile 4,6-Dichlor-5-hydroxy-2-phenylpyrimidin oder einer Mischung davon mit 2-Chlor-6'-äthyl-N-(2''-methoxy-1''-methyläthyl)-acet-o-toluidid,

10 Teile eines Alkylarylsulfonat/Fettalkoholpolyglykoläther-Gemisches,

5 Teile Dimethylformamid,

57,5 Teile Xylol.

Beispiel 10

Paste

Zur Herstellung einer 45%igen Paste werden folgende Stoffe verwendet:

a) 45 Teile 4,6-Dichlor-5-methoxy-2-phenylpyrimidin oder einer Mischung davon mit 2-Chlor-2',6'-diäthyl-N-(methoxymethyl)-acetanilid,

5 Teile Natriumaluminiumsilkat,

14 Teile Cetylpolyäthyleneglykoläther mit 8 Mol Aethylenoxid,

1 Teil Oleylpolyäthylenglykoläther mit 5 Mol Aethylenoxid,

2 Teile Spindelöl,

23 Teile Wasser,

10 Teile Polyäthylenglykol,

b) 45 Teile des obigen Wirkstoffes oder der Mischung,

5 Teile Aethylenglykol,

3 Teile Octylphenoxypolyäthylenglykol mit 9—10 Mol Aethylenoxid pro Mol Octylphenol,

3 Teile von einem Gemisch aromatischer Sulfonsulfosäuren, kondensiert mit Formaldehyd als Ammoniumsalz,

1 Teile Siliconöl in Form einer 75%igen Emulsion,

0,1 Teile einer Mischung von 1-(3-Chlorallyl)-3m5m7-triazo-azonium-adamantan-chlorid mit Natriumcarbonat, Chloridwert mind. 11,5%,

0,2 Teile eines biopolymeren Verdickers mit max. 100 Keimen pro Gramm,

42,7 Teile Wasser.

Die Aktivsubstanz wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

Biologische Beispiele

Die Fähigkeit der Verbindungen der Formel I, Kulturpflanzen vor der phytotoxischen Wirkung starker Herbizide zu schützen, kann aus dem folgenden Beispiel ersehen werden. In den Versuchsbeschreibungen werden die Verbindungen der Formel I als Antidote (Gegenmittel) bezeichnet. Die relative Schutzwirkung ist in % angegeben.

Beispiel 11

Versuch mit Antidote und Herbizid mit in Wasser gesätem Reis. Applikation der Antidote während der Samenquellung des Reises. Reissamen werden während 48 Stunden mit Lösungen der als Antidote zu prüfenden Substanz von 100 ppm getränkt. Anschliessend werden die Samen etwa 2 Stunden trocknen

gelassen, bis sie nicht mehr kleben. Plastik Container (25 cm lang, 17 cm breit und 12 cm hoch) werden bis 2 cm unter dem Rand mit sandigem Lehm gefüllt. Die vorgequollenen Samen werden auf der Bodenfläche des Containers gesät und mit wenig Erde gedeckt. Die Erde wird in einem feuchten (nicht sumpfigen) Zustand gehalten. Dann wird das Herbizid in verdünnter Lösung auf die Bodenoberfläche versprüht. Der Wasserstand wird entsprechend dem Wachstum sukzessive erhöht. 21 Tage danach wird die relative Schutzwirkung des Antidots in Prozent bonitiert. Als Referenzen dienen dabei die mit dem Herbizid allein behandelten Pflanzen (keine Schutzwirkung) sowie die vollständig unbehandelte Kontrolle (100% Wachstum). Die Ergebnisse sind in der untenstehenden Tabelle zusammengefasst.

Als Herbizid wird 2-Chlor-2',6'-diäthyl-N-(2''-propyloxyäthyl)-acetanilid ("Pretilachlor") in einer Aufwandmenge von 0,25 kg pro Hektar verwendet.

| Antidote No. | relative Schutzwirkung |
|---|---|
| 1 | 50 % |
| 3 | 12,5 % |
| 10 | 12,5 % |
| 11 | 12,5 % |
| 42 | 12,5 % |
| 60 | 38 % |
| 84 | 63 % |
| 94 | 50 % |
| 104 | 50 % |
| 105 | 63 % |
| 107 | 38 % |
| 108 | 50 % |
| 109 | 12,5 % |

Beispiel 12

Versuch mit Antidote und Herbizid mit in Wasser gesätem Reis. (Die Reissamen werden vorgequollen und direkt auf sehr nasse, sumpfartige oder überflutete Böden gesät. Applikation der Antidote als Tankmischung.

Reissamen werden während 48 Stunden vorgequollen. Plastik Container (25 cm lang, 17 cm breit und 12 cm hoch) werden mit Erde gefüllt, in die die vorgequollenen Reissamen eingesät werden. Anschliessend wird die als Antidote zu prüfende Substanz zusammen mit dem Herbizid als Tankmischung versprüht. Der Wassterstand wird entsprechend dem Wachstum der Reispflanzen sukzessive erhöht. 21 Tage danach wird die Schutzwirkung des Antidots in Prozent bonitiert. Als Referenzen dienen dabei die mit Herbizid allein behandelten Pflanzen, (keine Schutzwirkung) sowie die vollständig unbehandelte Kontrolle (100% Wachstum). Die Ergebnisse sind unten zusammengefasst.

Als Herbizid wird 2-Chlor-2'',6''-diäthyl-N-(2''-propyloxyäthyl)-acetanilid ("Pretilachlor") eingesetzt.

| Antidote Nr. | Aufwandmenge | Herbizid Aufwandmenge | relative Schutzwirkung |
|---|---|---|---|
| 1 | 1 kg /ha | 1 kg /ha | 63 % |
| 1 | 0,5 kg/ha | 0,5 kg/ha | 50 % |
| 84 | 1 kg /ha | 1 kg /ha | 25 % |
| 84 | 0,5 kg/ha | 0,5 kg/ha | 30 % |
| 103 | 1 kg/ha | 1 kg /ha | 25 % |
| 103 | 0,5 kg/ha | 0,5 kg/ha | 25 % |
| 104 | 0,5 kg/ha | 0,5 kg/ha | 25 % |
| 105 | 1 kg/ha | 1 kg/ha | 63 % |
| 105 | 0,5 kg/ha | 0,5 kg/ha | 63 % |
| 108 | 0,5 kg/ha | 0,5 kg/ha | 50 % |

Beispiel 13

Versuch mit Antidote und Herbizid in verpflanztem Reis. Applikation von Antidote und Herbizid als Tankmischung im Vorauflaufverfahren.

Reispflanzen werden bis zum 1 1/2-Blattstadium in Erde aufgezogen. Die Pflanzen werden dann büschelweise (immer 3 Pflanzen zusammen) in Container (47 cm lang, 29 cm breit und 24 cm hoch) in sandigem Lehm verpflanzt. Die Bodenoberfläche wird anschliessend mit Wasser von 1,5—2 cm Höhe beschichtet. 2—3 Tage nach dem Verpflanzen wird das Herbizid zusammen mit der als Antidote zu prüfenden Substanz als Tankmischung direkt ins Wasser appliziert. 24 Tage nach dem Verpflanzen wird die Schutzwirkung des Antidots in Prozent bonitiert. Als Referenzen dienen dabei die mit Herbizid allein behandelten Pflanzen (keine Schutzwirkung) sowie die vollständig unbehandelte Kontrolle (100% Wachstum)).

Die Resultate sind unten zusammengefasst.

Als Herbizid wird 2-Chlor-2″,6″-diäthyl-N-(2″-propyloxyäthyl)-acetanilid ("Pretilachlor") eingesetzt.

| Antidote Nr. | Aufwandmenge | Herbizid Aufwandmenge | relative Schutzwirkung |
|---|---|---|---|
| 1 | 1 kg /ha | 1 kg /ha | 38 % |
| 1 | 0,5 kg/ha | 1 kg /ha | 25 % |
| 1 | 0.75 kg/ha | 0.75 kg/ha | 38 % |
| 1 | 0.375 kg/ha | 0.75 kg/ha | 38 % |

Beispiel 14

Versuch mit Antidote und Herbizid in trocken gesätem Reis. Applikation des Antidotes als Samenbeize.

Reissamen werden mit der als Safener zu prüfenden Substanz in einen Glasbehälter gemischt. Samen und Produkt werden durch Schütteln und Rotation gut zusammengemischt. Dann werden Container (47 cm lang, 29 cm breit und 24 cm hoch) mit sandiger Erde gefüllt und die gebeizten Samen werden eingesät. Nach dem Bedecken des Samens wird das Herbizid in einer verdünnten Lösung auf die Bodenoberfläche versprüht. Etwa 20 Tage nach der Saat (3-Blattstadium der Reispflanzen) wird die Bodenoberfläche mit 4 cm Wasser Höhe beschichtet. 30 Tage nach der Herbizidapplikation wird die Schutzwirkung des Safeners in Prozent bonitiert. Als Referenzen dienen dabei die mit Herbizid allein behandelten Pflanzen (keine Schutzwirkung) sowie die vollständig unbehandelte Kontrolle (=Wachstum).

Die Resultate sind unten zusammengefasst.

*Herbizid:* 2-Chlor-2'-äthyl-6-methyl-N-(2''-methoxy-1''-methyläthyl)acetanilid ("Metalochlor").

| Antidote Nr. | Aufwandmenge | Herbizid Aufwandmenge | relative Schutzwirkung |
|---|---|---|---|
| 1 | 0,5 g/kg Samen | 0,5 kg/ha | 38 % |
| 1 | 0,5 g/kg Samen | 0,25 kg/ha | 25 % |

Beispiel 15

Versuch mit Antidote und Herbizid in Reis. Applikation des Antidotes als Samenbeize.

Reissamen werden mit der als Safener zu prüfenden Substanz in einen Glasbehälter gemischt. Samen und Produkt werden durch Schütteln und Rotation gut zusammengemischt. Anschliessend werden Plastikcontainer (47 cm lang, 29 cm breit und 24 cm hoch) mit sandiger Lehmerde gefüllt und die gebeizten Samen werden eingesät. Nach dem Bedecken des Samens wird das Herbizid auf die Bodenoberfläche versprüht. 18 Tage nach der Saat wird die Schutzwirkung des Safeners in Prozent bonitiert. Als Referenzen dienen dabei die mit Herbizid behandelten Pflanzen (keine Schutzwirkung) sowie die vollständig unbehandelte Kontrolle (=100% Wachstum).

Die Resultate sind unten zusammengefasst.

*Herbizid:* 2-Chlor-2'-Aethyl-6'-methyl-N-(1'-methoxy-1'-methyläthyl)acetanilid ("Metolachlor").

| Antidote Nr. | Aufwandmenge | Herbizid Aufwandmenge | relative Schutzwirkung |
|---|---|---|---|
| 1 | 0,5 g/kg Samen | 0,5 kg/ha | 38 % |
| 1 | 0,5 g/kg Samen | 0,25 kg/ha | 25 % |

Beispiel 16

Versuch mit Antidote und Herbizid in Sorghum (Hirse). Applikation von Herbizid und Antidote als Tankmischung im Vorauflaufverfahren.

Topfe, welche einen oberen Durchmesser von 6 cm haben, werden mit sandiger Lehmerde gefüllt und Sorghumsamen der Sorte G522 werden eingesät. Nach den Bedecken der Samen wird die als Safener zu prüfende Substanz zusammen mit dem Herbizid in verdünnter Lösung als Tankmischung auf die Bodenoberfläche versprüht. 21 Tage nach der Herbizidapplikation wird die Schutzwirkung des Safeners in Prozent bonitiert. Als Referenzen dienen dabei die mit Herbizid allein behandelten Pflanzen (keine Schutzwirkung) sowie die vollständig unbehandelte Kontrolle (=100% Wachstum).

Die Resultate sind unten zusammengefasst.

*Herbizid:* 2-Chlor-2'-äthyl-6'-methyl-N-(2''-methoxy-2''-methyläthyl)acetanilid ("Metolachlor").

| Antidote No. | Aufwandmenge | Herbizid Aufwandmenge | relative Schutzwirkung |
|---|---|---|---|
| 104 | 1,5 kg/ha | 1,5 kg/ha | 50 % |

**Patentansprüche**

1. 2-Aryl-4,6-dihalogenpyrimidine der allgemeinen Formel I

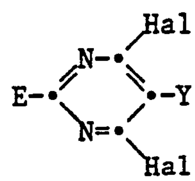

(I)

worin

E eine Gruppe

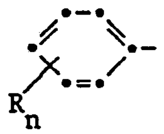 , 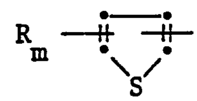 oder 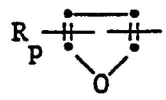 ,

Hal unabhangig voneinander Halogen und

Y ein Gruppe —$NR^1R^2$, —$OR^3$, —N=$CR^4R^5$, —N=CH—$NR^6R^7$, —SCN oder —N($R^4$)—CN bedeuten wobei

R Halogen, Nitro, Cyan, —$XR^8$, —$NR^9R^{10}$, —CO—$R^{11}$, —$COOR^{11}$, —CO—$NR^9R^{10}$, eine unsubstituierte oder durch Halogen, —$XR^8$, substituierte $C_1$—$C_6$-Alkylgruppe oder eine substituierte oder durch Halogen oder —$XR^8$ substituierte $C_2$—$C_6$-Alkenyl-, oder $C_2$—$C_6$-Alkinylgruppe,

n Null oder eine Zahl von eins bis drei,

m und p eine Zahl von Null bis zwei,

$R^1$, $R^2$ und $R^3$ unabhängig voneinander Wasserstoff, —$COR^4$, —$COOR^4$, —$CON(R^4)_2$, —$CONHR^{12}$, $C_3$—$C_6$-Alkoxy substituiertes $C_1$—$C_6$-Alkyl oder $C_3$—$C_6$-Alkenyl oder

$R^8$ Wasserstoff, $C_1$—$C_6$-Alkylcarbonyl, oder eine unsubstituierte $C_1$—$C_6$-Alkyl- gruppe,

$R^9$ und $R^{10}$ unabhängig voneinander Wasserstoff, oder unsubstituiertes Alkyl, wobei auch einer der beiden Reste für —$OR^{11}$, $COOR^{11}$, —CO—$N(R^{11})_2$ oder —CO—$N(R^{11})$—$OR^{11}$ stehen kann,

$R^{11}$ Wasserstoff, eine unsubstituierte oder durch Halogen, substituierte $C_1$—$C_6$-Alkyl, $C_3$—$C_6$-Alkenyl odr $C_3$—$C_6$-Alkinylgruppe,

$R^{12}$ einen Phenylrest

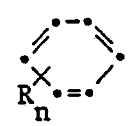

und

mit der Massgabe, dass wenn E ein unsubstituiertes Phenyl und Hal Chlor b eutet, in einem Aminorest Y eines von $R^1$ und $R^2$ nicht Wasserstoff, —$COOR^4$ oder —$COHNR^{12}$ sein kann, wenn das andere Wasserstoff ist.

2. Verbindung gemäss Anspruch 1, dadurch gekennzeichnet, dass E für

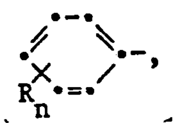

worin R und n die in Anspruch 1 gegebene Bedeutung haben, steht.

3. Verbindungen gemäss Anspruch 1, dadurch gekennzeichnet, dass E für eine der Gruppen

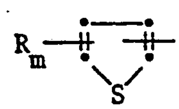 oder 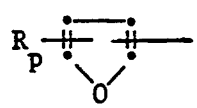

4. Verbindungen gemäss Anspruch 1, dadurch gekennzeichnet, dass E für einen Phenylrest

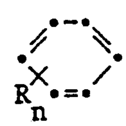

steht, p eine Zahl von Null bis zwei und R Halogen, Nitro, Cyan, —$XR^8$, $NR^9R^{10}$, —CO—$R^{11}$, —CO—$NR^9R^{10}$, $C_1$—$C_4$-Alkyl, $C_2$—$C_4$-Alkenyl oder $C_2$—$C_4$-Alkinyl bedeuten, wobei n, R, $R^8$, $R^9$, $R^{10}$ und $R^{11}$ die in Anspruch 1 gegebene Bedeutung haben.

5. Verbindungen gemäss Anspruch 1, dadurch gekennzeichnet, dass E für einen unsubstituierten oder einfach durch Methoxy, Fluor, Hydroxyl, Methyl, Aethinyl, Carboxyl, Acetyl, Nitro, Amino, Dimethylamino, Acetamido oder Methylaminocarbonyloxy oder zweifach durch Fluor oder Chlor oder dreifach durch Methoxy substituierten Phenylkern steht, Hal für Chlor oder Brom steht und Y —$NR^1R^2$, —$OR^3$, —$SR^3$, —N=N—$NR^6R^7$ oder —N=$CHR^4R^5$ steht, wobei $R^1$ bis $R^7$ die unter Anspruch 1 gegbebene Bedeutung haben.

6. Verbindungen gemäss Anspruch 1, dadurch gekennzeichnet, dass E für einen unsubstituierten oder einfach durch Methoxy, Fluor, Hydroxyl, Methyl, Aethinyl, Carboxyl, Acetyl, Nitro, Amino, Dimethylamino, Acetamido oder Methylaminocarbonyloxy oder zweifach durch Fluor oder Chlor oder dreifach durch Methoxy substituierten Phenylkern steht, Hal für Chlor oder Brom steht und Y für —$NR_1R_2$ steht, wobei $R_1$ und $R_2$ die unter Anspruch 1 gegebene Bedeutung haben.

7. Verbindungen gemäss Anspruch 1, dadurch gekennzeichnet, dass E für Phenyl, Hal für Chlor und Y für —$NR_1R_2$ stehen, wobei $R_1$ und $R_2$ stehen, wobei $R_1$ und $R_2$ die unter Anspruch 1 gegebene Bedeutung haben.

8. Verbindungen gemäss Anspruch 1, dadurch gekennzeichnet, dass E für Phenyl, Hal für Chlor und Y für Dimethylamino, Diacetamido, Trifluoracetamido, Hydroxyl, Methoxy, Methylthio, Acetoxy, Acetamido oder Methylaminocarbonyloxy stehen.

9. 5-Amino-4,6-dichlor-2-(3'-methoxyphenyl)-pyrimidin gemäss Anspruch 1.

10. 5-(N,N-Di-(phenoxycarbamoyl)-amino-4,6-dichlor-2-phenylpyrimidin gemäss Anspruch 1.

11. 5-Amino-4,6-dibrom-2-phenylpyrimidin gemäss Anspruch 1.

12. Verfahren zur Herstellung der 2-Aryl-4,6-dihalogenpyrimidine der Formel I, gemäss Anspruch 1, dadurch gekennzeichnet, dass man ein Arylamidin der Formel II

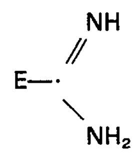

(II)

worin E die unter Formel I gegebene Bedeutung hat, in Gegenwart einer Base mit einem Malonsäurederivat der Formel III

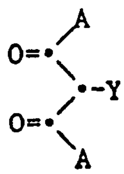

(III),

worin Y die unter Formel I gegebene Bedeutung hat und A für Amino oder $C^1$—$C_6$-Alkoxy steht, umsetzt und das erhaltene, 4,6-Dihydroxypyrimidin der Formel IV

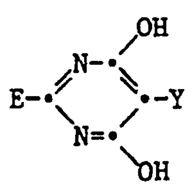

(IV),

worin E und Y die unter Formel I gegebene Bedeutung haben, mit einem Halogenierungsmittel in die Verbindung der Formel I Überführt.

13. Mittel zum Schützen von Kulturpflanzen vor Schädigung durch Herbizide, dadurch gekennzeichnet dass es neben inerten Träger- und Zusatzstoffen als wirksame Komponente ein 2-Aryl-4,6-dihalogenpyrimidin der Formel I gemäss Anspruch 1 enthält.

14. Mittel nach Anspruch 13, gekennzeichnet durch einen Gehalt an

a) einen herbizid wirksamen Chloracetanilid oder anderen herbizid wirksamen Stoff und

b) ein 2-Aryl-4,6-dihalogenpyrimidin der Formel I gemäss Anspruch 1 als Gegenmittel.

15. Verfahren zur Verwendung der 2-Aryl-4,6-dihalogenpyrimidine der Formel I gemäss Anspruch 1 zum Schützen von Kulturpflanzen gegen die schädigende Wirkung von Herbiziden, dadurch gekennzeichnet, dass man eine wirksame Menge der Wirkstoffe auf die Pflanzen, ihre Samen oder ihren Lebensraum appliziert.

16. Verfahren gemäss Anspruch 15 zum Schützen von Getreide, Reis-, Mais- und Sorghum-Kulturen vor der Schädigung durch Chloracetanilid-Herbizide oder andere herbizid wirksamen Stoffe.

17. Verfahren gemäss Anspruch 16 zum Schützen von Reis vor der Schädigung durch Chloracetanilid-Herbizide.

18. Verfahren gemäss Anspruch 16 zum Schützen von Kulturpflanzen vor Schäden, die bei der Applikation von Herbiziden auftreten, dadurch gekennzeichnet, dass man

a) die Anbaufläche für die Pflanze vor oder während der Applikation des Herbizids oder

b) den Samen oder die Stecklinge der Pflanzen oder die Pflanze selbst mit einer wirksamen Menge eines 2-Aryl-4,6-dihalogenpyrimidins der Formel I gemäss Anspruch 1, behandelt.

19. Saatgut von Kulturpflanzen, welches mit einer wirksamen Menge eines 2-Acyl-4,6-dihalogenpyrimidines der Formel I, Anspruch 1, behandelt sind.

## Revendications

1. 2-aryl-4,6-dihalogénopyrimidines de formule générale I

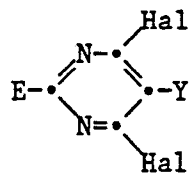

(I)

dans laquelle

E représente un groupe

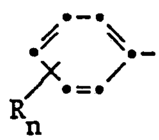 , 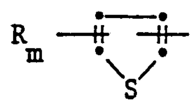 ou 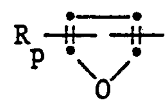 ,

les symboles Hal représentent chacun, indépendamment l'un de l'autre, un halogène et

Y représente un groupe $—NR^1R^2$, $—OR^3$, $—N{=}CR^4R^5$, $—N{=}CH—NR^6R^7$, —SCN ou $—N(R^4)—CN$ dans lesquels

R représente un halogène, un groupe nitro, cyano, $—XR^8$, $—NR^9R^{10}$, $—CO—R^{11}$, $—COOR^{11}$, $—CO—NR^9R^{10}$, un groupe alkyle en C1—C6 non substitué ou substitué par des halogènes ou des groupes $—XR^8$, ou un groupe alcényle en C2—C6 ou alcynyle en C2—C6 non substitué ou substitué par des halogènes ou des groupes $—XR^8$,

n est égal à O ou représente un nombre allant de 1 à 3,

m et p sont des nombres allant de 0 à 2,

$R^1$, $R^2$ et $R^3$ représentent chacun, indépendamment les uns des autres, l'hydrogène, un groupe $—COR^4$, $COOR^4$, $—CON(R^4)_2$, $—CONHR^{12}$, alcynyle en C3—C6 ou un groupe alkyle en C1—C6 ou alcényle en $C^3$—C6 non substitué ou substitué par des halogènes ou des groupes alcoxy en C1—C4,

ou bien

$R^8$ représente l'hydrogène, un groupe (alkyle en C1—C6)-carbonyle ou un groupe alkyle en C1—C6 non substitué,

$R^9$ et $R^{10}$ représentent chacun, indépendamment l'un de l'autre, l'hydrogène ou un groupe alkyle non substitué, l'un de ces deux symboles pouvant également représenter $—OR^{11}$, $—COOR^{11}$, $—CO—N(R^{11})_2$ ou $—CO—N(R^{11})—OR^{11}$,

$R^{11}$ représente l'hydrogène, un groupe alkyle en C1—C6, alcényle en C3—C6 ou alcynyle en C3—C6 non substitué ou substitué par des halogènes,

$R^{12}$ représente un groupe phényle

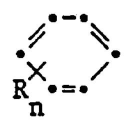

sous réserve que, lorsque E représente un groupe phényle non substitué et Hal le chlore, l'un des symboles $R^1$ et $R^2$ d'un groupe amino Y ne peut représenter l'hydrogène, $—COOR^4$ ou $—CONHR^{12}$ lorsque l'autre représente l'hydrogène.

2. Composé selon la revendication 1, caractérisés en ce que E représente

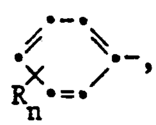

R et n ayant les significations indiquées dans la revendication 1.

3. Composés selon la revendication 1, caractérisés en ce que E représente l'un des groupes

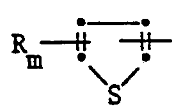 ou 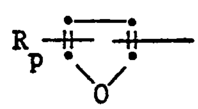

dans lesquels R a les significations indiquées dans la revendication 1 et m et p sont des nombres allant de 0 à 2.

4. Composés selon la revendication 1, caractérisés en ce que E représente un groupe phényle

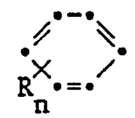

p est un nombre allant de 0 à 2 et R représente un halogène, un groupe nitro, cyano, —$XR^8$, $NR^9R^{10}$, —CO—$R^{11}$, —CO—$NR^9R^{10}$, alkyle en C1—C4, alcényle en C2—C4 ou alcynyle en C2—C4, et n, R, $R^8$, $R^9$, $R^{10}$ et $R^{11}$ ont les significations indiquées dans la revendication 1.

5. Composés selon la revendication 1, caractérisés en ce que E représente un noyau phényle non substitué ou mono-substitué par un groupe méthoxy, le fluor, un groupe hydroxy, méthyle, éthynyle, carboxyle, acétyle, nitro, amino, diméthylamino, acétamido ou méthylaminocarbonyloxy, ou disubstitué par le fluore ou le chlore, ou trisubstitué par des groupes méthoxy, Hal représente le chlore ou le brome et Y représente —$NR^1R^2$, —$OR^3$, —$SR^3$, —$SR^3$, —N=N—$NR^6R^7$ ou —N=$CHR^4R^5$ dans lesquels $R^1$ à $R^7$ ont les significations indiquées dans la revendication 1.

6. Composés selon la revendication 1, caractérisés en ce que E représente un noyau phényle non substitué ou mono-substitué par un groupe méthoxy, le fluor, un groupe hydroxy, méthyle, éthynyle, carboxyle, acétyle, nitro, amino, diméthylamino, acétamido ou méthylaminocarbonyloxy, ou disubstitué par le fluore ou le chlore, ou trisubstitué par des groupes méthoxy, Hal représente le chlore ou le brome et Y représente —$NR^1R^2$, $R^1$ et $R^2$ ayant les significations indiquées dans la revendication 1.

7. Composés selon la revendication 1, caractérisés en ce que E représente un groupe phényle, Hal représente le chlore et Y représente —$NR^1R^2$, $R^1$ et $R^2$ ayant les significations indiquées dans la revendication 1.

8. Composés selon la revendication 1, caractérisés en ce que E représente un groupe phényle, Hal représente le chlore et Y un groupe diméthylamino, diacétamido, trifluoroacétamido, hydroxy, méthoxy, méthylthio, acétoxy, acétamido ou méthylaminocarbonyloxy.

9. La 5-amino-4,6-dichloro-2-(3'-méthoxyphényl)-pyrimidine selon la revendication 1.

10. La 5-N,N-di(phénoxycarbamoyl)-amino-4,6-dichloro-2-phénylpyrimidine selon la revendication 1.

11. La 5-amino-4,6-dibromo-2-phénylpyrimidine selon la revendication 1.

12. Procédé de préparation des 2-aryl-4,6-dihalogénopyrimidines de formule I de la revendication 1, caractérisé en ce que l'on fait réagir une arylamidine de formule II

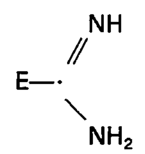

(II)

dans laquelle E a les significations indiqués en référence à la formule I, en présence d'une base, avec un dérivé de l'acide malonique répondant à la formule III

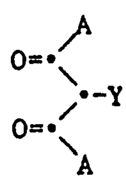

(III),

dans laquelle Y a les significations indiquées en référence à la formule I et A représente un group amino ou alcoxy en C1—C6, ce qui donne une 4,6-dihydroxypyrimidine de formule V

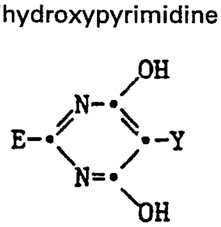

(IV),

dans laquelle E et Y ont les significations indiquées en référence à la formule I, qu'on convertit en le composé de formule I à l'aide d'un agent halogénant.

13. Produit pour protéger les végétaux cultivés contre les dommages provoqués par des herbicides, caractérisé en ce qu'il contient, en tant que composant actif, avec des véhicules et additifs inertes, une 2-aryl-4,6-dihalogénopyrimidine de formule I de la revendication 1.

14. Produit selon la revendication 13, caractérisé en ce qu'il contient

a) un chloroacétanilide possédant une activité herbicide ou une autre substance possédant une activité herbicide et

b) un 2-aryl-4,6-dihalogénopyrimidine de formule I de la revendication 1, en tant qu'antidote.

15. Procédé pour utiliser les 2-aryl-4,6-dihalogénopyrimidines de formule I de la revendication 1 pour la protection des végétaux cultivés contre les effets nocifs des herbicides, caractérisé en ce que l'on applique une quantité efficace de ces substances actives sur les végétaux, leurs semences ou leur habitat.

16. Procédé selon la revendication 15, pour protéger les cultures de céréales, de riz, de maïs et de sorgho contre les dommages provoquées par les herbicides du type chloroacétanilide ou d'autres substances possédant une activité herbicide.

17. Procédé selon la revendication 16 pour protéger le riz contre les dommages provoquées par les herbicides du type chloroacétanilide.

18. Procédé selon la revendication 16 pour protéger las végétaux cultivés contre les dommages survenant à l'application d'herbicides, caractérisé en ce que

a) on traite l'aire de culture de la plante, avant ou après l'application de l'herbicide, ou bien

b) on traite les semences ou les pousses de la plante ou la plante elle-même par une quantité efficace d'une 2-aryl-4,6-dihalogénopyrimidine de la formule I de la revendication 1.

19. Semences de végétaux cultivés, traitées par une quantité efficace d'une 2-aryl-4,6-dihalogénopyrimidine de formula I, revendication 1.

**Claims**

1. A 2-aryl-4,6-dihalopyrimidine of the general formula I

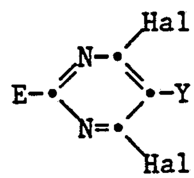

(I)

in which

E is a group

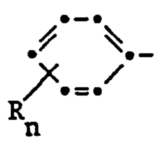 , 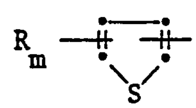 or 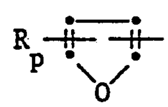 ,

Hal each independently of the other is halogen, and

Y is a group: $-NR^1R^2$, $-OR^3$, $-N=CR^4R^5$, $-N=CH-NR^6R^7$, $-SCN$ or $-N(R^4)-CN$, in which

R is halogen, nitro, cyano, $-XR^8$, $-NR^9R^{10}$, $-CO-R^{11}$, $-COOR^{11}$, $-CO-NR^9R^{10}$, a $C_1-C_6$alkyl group which is unsubstituted or substituted by halogen or $-XR^8$, or it is a $C_2-C_6$alkenyl or $C_2-C_6$alkynyl group each unsubstituted or substituted by halogen or $-XR^8$,

n is zero or a number from one to three,

m and p are a number from zero to two,

$R^1$, $R^2$ and $R^3$ independently of one another are each hydrogen, $-COR^4$, $-COOR^4$, $-CON(R^4)_2$, $-CONHR^{12}$, $C_3-C_6$alkynyl or $C_1-C_6$alkyl or $C_3-C_6$alkenyl each unsubstituted or substituted by halogen or $C_1-C_4$alkoxy, or

$R^8$ is hydrogen, $C_1-C_6$alkylcarbonyl, or an unsubstituted $C_1-C_6$alkyl group,

$R^9$ and $R^{10}$ independently of one another are each hydrogen, or unsubstituted alkyl, and also one of the two radicals can be $-OR^{11}$, $-COOR^{11}$, $-CO-N(R^{11})_2$ or $-CO-N(R^{11})-OR^{11}$,

$R^{11}$ is hydrogen, or a $C_1-C_6$alkyl, $C_3-C_6$alkenyl or $C_3-C_6$alkynyl group each unsubstituted or substituted by halogen,

$R^{12}$ is a phenyl radical

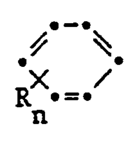

with the proviso that when E is an unsubstituted phenyl and Hal is chlorine, one of $R^1$ and $R^2$ in an amino radical Y cannot be hydrogen, $-COOR^4$ or $-CONHR^{12}$ if the other is hydrogen.

2. A compound according to claim 1, wherein E is

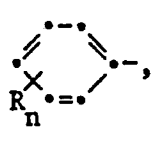

in which R and n have the meanings defined in claim 1.

3. A compound according to claim 1, wherein E is one of the groups

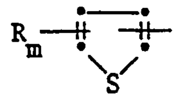 or 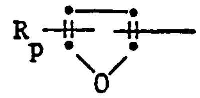

in which R has the meaning defined in claim 1, and m and p are numbers from zero to two.

4. A compound according to claim 1, wherein E is a phenyl radical

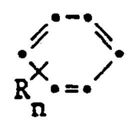

p is a number from zero to two, and R is halogen, nitro, cyano, $-XR^8$, $-NR^9R^{10}$, $-CO-R^{11}$, $-CO-NR^9R^{10}$, $C_1-C_4$alkyl, $C_2-C_4$alkenyl or $C_2-C_4$alkynyl, and n, R, $R^8$, $R^9$, $R^{10}$ and $R^{11}$ have the meanings defined in claim 1.

5. A compound according to claim 1, wherein E is a phenyl nucleus which is unsubstituted or monosubstituted by methoxy, fluorine, hydroxyl, methyl, ethynyl, carboxyl, acetyl, nitro, amino, dimethylamino, acetamido or methylaminocarbonyloxy, or is disubstituted by fluorine or chlorine, or is trisubstituted by methoxy, Hal is chlorine or bromine, and Y is $-NR^1R^2$, $-OR^3$, $-SR^3$, $-N=N-NR^6R^7$ or $-N=CHR^4R^5$, $R^1$ to $R^7$ having the meanings defined in claim 1.

6. A compound according to claim 1, wherein E is a phenyl nucleus which is unsubstituted or monosubstituted by methoxy, fluorine, hydroxyl, methyl, ethynyl, carboxyl, acetyl, nitro, amino, dimethylamino, acetamido or methylaminocarbonyloxy, or is disubstituted by fluorine or chlorine, or is trisubstituted by methoxy, Hal is chlorine or bromine, and Y is $-NR_1R_2$, $R_1$ and $R_2$ having the meanings defined in claim 1.

7. A compound according to claim 1, wherein E is phenyl, Hal is chlorine, and Y is $-NR_1R_2$, $R_1$ and $R_2$ having the meanings defined in claim 1.

8. A compound according to claim 1, wherein E is phenyl, Hal is chlorine, and Y is dimethylamino, diacetamido, trifluoroacetamido, hydroxyl, methoxy, methylthio, acetoxy, acetamido or methylaminocarbonyloxy.

9. 5-Amino-4,6-dichloro-2-(3'-methoxyphenyl)-pyrimidine according to claim 1.

10. 5-(N,N-Di-(phenoxycarbamoyl)-amino-4,6-dichloro-2-phenylpyrimidine according to claim 1.

11. 5-Amino-4,6-dibromo-2-phenylpyrimidine according to claim 1.

12. A process for producing a 2-aryl-4,6-dihalopyrimidine of the formula I according to claim 1, which process comprises reacting an arylamidine of the formula II

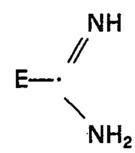 (II)

in which E has the meaning defined under the formula I, in the presence of a base with a malonic acid derivative of the formula III

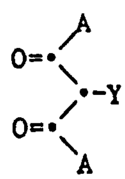 (III),

in which Y has the meaning defined under the formula I, and A is amino or $C_1-C_6$alkoxy, and subsequently converting the resulting 4,6-dihydroxypyrimidine of the formula IV

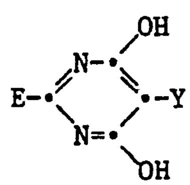 (IV),

in which E and Y have the meanings defined under the formula I, with a halogenating agent into the compound of the formula I.

13. A composition for protecting cultivated plants against damage by herbicides, which composition contains an as active component a 2-aryl-4,6-dihalopyrimidine of the formula I according to claim 1, together with inert carriers and additives.

14. A composition according to claim 13, which contains

a) a herbicidally effective chloroacetanilide or some other herbicidally effective substance, and

b) a 2-aryl-4,6-dihalopyrimidine of the formula I according to claim 1 as antidote.

15. A method in which a 2-aryl-4,6-dihalopyrimidine of the formula I according to claim 1 is used for protecting cultivated plants against the harmful action of herbicides, which method comprises applying an effective amount of the active ingredients to the plants, to the seeds of the plants, or to the locus thereof.

16. A method according to claim 15 for protecting cereals, rice, maize, and sorghum crops against damage by chloroacetanilide herbicides or other herbicidally effective substances.

17. A method according to claim 16 for protecting rice plants against damage by chloroacetanilide herbicides.

18. A method according to claim 16 for protecting cultivated plants against damage which occurs on application of herbicides, which method comprises

e) treating the cultivated area for the plants before or during application of the herbicide, or

b) treating the seeds or the seedlings of the plants or the plant itself with an effective amount of a 2-aryl-4,6-dihalopyrimidine of the formula I according to claim 1.

19. Seed of cultivated plants which has been treated with an effective amount of a 2-acyl-4,6-dihalopyrimidine of the formula I, claim 1.